# FASCICULE DE BREVET EUROPEEN

(11) **EP 2 438 929 B1**
(45) Date de publication et mention de la délivrance du brevet: **10.06.2020**
(21) Numéro de dépôt: 11177669.6
(22) Date de dépôt: 24.01.2007
(51) Int. Cl.: A61K 31/5395, A61K 9/00

(54) **Solution anti-infectieuse comprenant un composé pyrido(3,2,1-ij)-benzoxadiazine**
Antiinfektiöse Lösung enthaltend eine Pyrido-(3,2,1-ij)-benzoxadiazinverbindung
Anti-infective solution comprising a pyrido(3,2,1-ij)-benzoxadiazine compound

(30) Priorité: 24.01.2006 FR 0650246
(43) Date de publication de la demande: 11.04.2012
(62) Demande divisionnaire de: 07731510.9
(73) Titulaire: SA VETOQUINOL, 70200 Lure (FR)
(72) Inventeur: Moreau, Marinette, 70200 Saint Germain (FR)
(74) Mandataire: Novagraaf Technologies

(56) Documents cités:
- EP-A2- 0 219 784
- WO-A1-2005/049081
- DE-A1- 19 500 784
- US-A- 6 103 716
- ANONYMOUS: "Vetoquinol AG", , septembre 2005 (2005-09), XP002444299, Extrait de l'Internet: URL:http://www.vetpharm.uzh.ch/reloader.ht m?http://www.vetpharm.uzh.ch/TAK/05000000/ 00055101.02?inhalt_c.htm [extrait le 2007-07-26]
- Pfizer Inc: "Zeniquin (marbofloxacin) Tablets", , 1 August 2001 (2001-08-01), pages 1-28, XP055364999, US Retrieved from the Internet: URL:https://www.fda.gov/ohrms/dockets/98fr /141151fi.pdf [retrieved on 2017-04-18]

## Description

Le domaine technique de la présente invention concerne les solutions anti-infectieuses destinées à traiter les animaux.

La présente invention concerne plus précisément une solution aqueuse anti-infectieuse injectable comprenant au moins un composé pyrido(3,2,1-ij)-benzoxadiazine, et en particulier une solution concentrée.

Chez les animaux de rente, comme les bovins, porcins, caprins, ovins, volailles et/ou cheval, ainsi que chez les animaux de compagnie, l'administration, notamment par injection, de produits à visés thérapeutiques, prophylactiques ou métaphylactiques à visées anti-infectieuse est fréquente.

Le volume de produit administré par voie parentérale ainsi que le nombre de sites d'injection sont des critères importants dans le choix d'une formulation. Un objectif de la réalisation d'une formule concentrée est de diminuer le volume et/ou le nombre d'administrations, notamment en vue de réduire, pour les animaux de rente, les zones musculaires non valorisées à l'abattoir. Un autre critère important, qui peut également être pris en compte dans la réalisation de formules concentrées, est la tolérance locale, au site d'injection, notamment pour réduire les pertes à l'abattoir.

Cependant certains des produits anti-infectieux, par exemple la Marbofloxacine, présentent une faible solubilité dans l'eau. Ceci peut conduire à utiliser des technologies permettant d'augmenter leur solubilité, comme la salification, l'utilisation de complexes plus solubles que la molécule elle-même, par exemple en combinaison avec une cyclodextrine, de modificateurs de pH, de systèmes dispersés (émulsions, liposomes, systèmes vectoriels), ou l'ajout d'adjuvants tels les solvants organiques, les co-solvants, ou les tensioactifs.

Cependant, les techniques évoquées ci-dessus peuvent présenter une efficacité très relative par rapport à certains composés spécifiques.

Parmi les solutions commercialisées, on peut citer des solutions stables contenant jusqu'à 10 % de Marbofloxacine. Cependant, pour certaines indications thérapeutiques, prophylactiques ou métaphylactiques, notamment chez les animaux de rente, la dose thérapeutique est élevée. Ainsi l'emploi des solutions actuellement commercialisées nécessite l'administration de grands volumes d'injection, notamment supérieurs ou égaux à 40 ml, répartis en plusieurs sites d'injection. Ces conditions peuvent conduire à des problèmes de tolérance.

Le document EP 0 868 183 décrit des solutions aqueuses pharmaceutiques concentrées de danofloxacine comprenant des sels métalliques, dont la tolérance, au site d'injection, serait améliorée.

Cependant ce type de formulation, précédemment décrit pour les tétracyclines peut se révéler ne pas être applicable ou en tout cas ne pas être suffisamment efficace par rapport à tout type de molécule, en particulier en ce qui concerne certaines quinolones.

Or, pour différentes raisons, dont l'amélioration du bien être animal, un gain de temps, et/ou l'amélioration du rendement en viande, il peut être souhaitable de diminuer le volume de produit administré par voie parentérale, le nombre de sites d'injection et/ou d'améliorer la tolérance.

Il existe donc toujours un besoin pour des formules concentrées de composés anti-infectieux, en particulier de type quinolone, permettant de diminuer le volume, le nombre d'administrations et/ou présentant une tolérance améliorée, pouvant ainsi, pour les animaux de rente, diminuer les zones musculaires non valorisables.

Selon un de ses aspects, l'invention a pour objet une solution aqueuse anti-infectieuse injectable comprenant (i) de 15 à 20% en poids par rapport au volume total de la solution d'au moins un composé de type pyrido(3,2,1-ij)-benzoxadiazine, ou au moins un de ses sels pharmaceutiquement acceptables, (ii) un agent stabilisant choisi dans le groupe comprenant les alcools, les propylènes glycol, le polyéthylène glycol et la glycérine, et (iii) au moins un agent de solubilisation ou agent solubilisant choisi dans le groupe comprenant les acides minéraux, les acides organiques et leurs esters, et leurs mélanges, dans un support pharmaceutiquement acceptable.

Par « composé de type pyrido(3,2,1-ij)-benzoxadiazine », on entend un composé répondant à la formule (I) suivante : 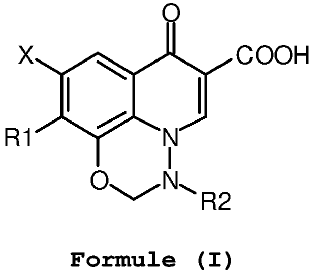 dans laquelle :
- X représente un atome d'hydrogène, d'halogène ou une fonction hydroxy, et en particulier un atome de fluor,
- R1 représente :
   - un radical 1-pipérazinyle, qui peut être substitué en position 4 par un groupe méthyle, acétyle ou 4-aminobenzyle ;
   - un radical morpholino ;
   - un radical 1-pyrrolidinyle substitué en position 3 par un atome de chlore ou par un groupe amino, aminométhyle, (méthylamino) méthyle, (éthylamino) méthyle ou méthoxy ;
   - un radical 1-imidazolyle qui peut être substitué en position 4 par le groupe méthyle ou
   - un radical 1-pipéridyle substitué en position 4 par un groupe hydroxy ou méthoxy, et
- R2 représente un radical alkyle, linéaire, ramifié ou cyclique, comprenant de 1 à 10 atomes de carbone, notamment un groupe méthyle, et
ses sels pharmaceutiquement acceptables.

Le composé de type pyrido(3,2,1-ij)-benzoxadiazine peut tout particulièrement répondre à la formule (I) dans laquelle R1 représente un radical 1-pipérazinyle substitué en position 4 par un groupe méthyle, R2 représente un groupement méthyle et X représente un atome de fluor. Ce composé peut notamment être obtenu par les procédés décrits dans le document EP 0 259 804. Ce composé répond notamment à la Dénomination Commune Internationale de Marbofloxacine.

La solution comprend au moins un agent de solubilisation. Cet agent de solubilisation peut former un complexe avec le composé de type pyrido(3,2,1-ij)-benzoxadiazine. En particulier lorsque cet agent de solubilisation est un acide, il peut former un sel avec le composé de type pyrido(3,2,1-ij)-benzoxadiazine.

La formation de tels complexes peut permettre d'améliorer la solubilisation du composé de type pyrido(3,2,1-ij)-benzoxadiazine en solution, et en particulier en solution aqueuse.

Parmi les agents de solubilisation, on peut citer :
- les acides minéraux, notamment l'acide chlorhydrique, bromhydrique, sulfurique, phosphorique et nitrique, et
- les acides organiques, comme les acides carboxyliques les acides sulfoniques et les acides phosphoniques ; notamment l'acide formique, acétique, propionique, succinique, glycolique, lactique ou polylactique, malique, tartrique, citrique, ascorbique, maléique, hydroxymaléique, phénylacétique, benzoïque, 4-aminobenzoïque, anthranilique, 4-hydroxybenzoïque, salicylique, aminosalicylique, nicotinique, méthane sulfonique, éthane sulfonique, hydroxyéthane sulfonique, benzène sulfonique, p-toluènesulfonique ; les acides polycarboxyliques, comme l'acide gluconique, l'acide glucuronique, l'acide galacturonique, l'acide isophtalique et l'acide lactobionique ; les acides aminés, comme l'acide aspartique et glutamique, la méthionine, le tryptophane, la lysine, l'arginine ; leurs esters, parmi lesquels on peut citer la gluconolactone ; et leurs sels, et
- leurs mélanges.

Parmi les agents de solubilisation des composés de type pyrido(3,2,1-ij)-benzoxadiazine, et en particulier la Marbofloxacine, on peut tout particulièrement citer l'acide lactique, l'acide gluconique et la gluconolactone.

La solution peut comprendre une teneur en agent de solubilisation allant de 4 à 58 % en poids, notamment de 5 à 35 % en poids, en particulier allant de 6 à 25 % en poids, voire allant de 7 à 20 % en poids par rapport au volume total de la solution.

Tout particulièrement, la solution selon l'invention comprend un rapport molaire agent de solubilisation/composé de type pyrido(3,2,1-ij)-benzoxadiazine allant de 0,9 à 4, notamment de 1 à 3, et en particulier de 1,1 à 2,4.

La solution peut comprendre une teneur en agent de solubilisation telle que le pH de la solution va de 2 à 7, notamment de 2,8 à 5.

Les solutions décrites ci-dessus peuvent présenter une stabilité limitée, notamment lorsqu'elles comprennent une forte concentration en composé de type pyrido(3,2,1-ij)-benzoxadiazine et une faible concentration en agent de solubilisation.

Les inventeurs ont découvert qu'il était possible d'améliorer la faisabilité de certaines solutions concentrées et/ou leur stabilité par l'addition d'au moins un agent solubilisant et/ou d'au moins un agent stabilisant.

L'agent stabilisant peut être choisi dans le groupe comprenant les alcools, les propylènes glycol, le polyéthylène glycol, la glycérine. Plus particulièrement, l'agent stabilisant est un alcool, notamment aromatique, alkyle, arylalkyle ou alkylaryle, en particulier comprenant de 5 à 15 atomes de carbone, et tout spécialement l'alcool benzylique et ses dérivés.

On entend par « dérivé d'alcool benzylique » au sens de la présente invention des composés répondant à la formule (II) : 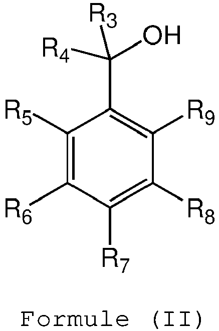 dans laquelle R₃, R₄, R₅, R₆, R₇, R₈ et R₉ représentent indépendamment un atome d'hydrogène, d'halogène, une fonction alcool, éther, amine, éventuellement substituée, thioéther, ester, amide, éventuellement substituée, un radical alkyle, aryle, aralkyle, alkaryle.

En particulier, la solution comprend une teneur en agent stabilisant allant de 0,2 à 20 % en poids, notamment de 0,3 à 10 % en poids, en particulier de 0,4 à 5 % en poids, et tout particulièrement de 0,5 à 3 % en poids par rapport au volume total de la solution.

D'autre part, la solution peut comprendre un rapport molaire agent stabilisant/composé de type pyrido(3,2,1-ij)-benzoxadiazine allant de 0,02 à 7, notamment de 0,05 à 5, en particulier de 0,1 à 1,5, voire de 0,2 à 1.

En particulier, les solutions peuvent comprendre un rapport molaire agent solubilisant/agent stabilisant allant de 1 à 15, notamment de 1,5 à 10, en particulier de 2 à 8, voire de 2,5 à 7.

Les solutions selon l'invention peuvent en outre comprendre au moins un additif, notamment choisi dans le groupe comprenant :
- un solvant, par exemple choisi parmi les solvants non aqueux utilisables par voie parentérale i.e. les solvants non miscibles à l'eau, par exemple les huiles végétales, l'oléate d'éthyle, ou les solvants miscibles à l'eau, notamment les alcools, comme l'éthanol ; les amides, comme le N,N diméthylacétamide ; les esters de polyol, comme le glycéride polyglucosé ; les éthers, comme le diméthyl-1,3-dioxolane-4-méthanol, l'éther monoéthylique du diéthylène glycol, le glycérol formal, le polyéthylène glycol, notamment PEG 300 et PEG 400 ; les polyols, comme le glycérol, le propylène glycol ; le diméthylsulfoxyde ; le 2-pyrrolidone ; ou un mélange de ceux-ci,
- un tensioactif, par exemple non ionique, anionique, cationique, ou un mélange de tensioactif, et tout particulièrement un tensioactif non ionique,
- un agent chélatant, par exemple l'EDTA
- un conservateur, notamment un antioxydant, ou un antimicrobien, et
- un mélange de ceux-ci.

Le pH de la solution peut aller de 2 à 7, notamment de 2,8 à 5.

Tout particulièrement, la solution selon l'invention est sous forme d'une solution injectable, en particulier par voie intramusculaire, sous-cutanée, intrapéritonéale et/ou intraveineuse. Ladite solution peut encore être une infusion ou une perfusion. Cette solution peut être destinée aux animaux dits de rente, comme les ovins, bovins, porcins, caprins, chevaux et/ou volailles, et/ou aux animaux de compagnie, comme les chiens et les chats.

Tout particulièrement, la solution présente une viscosité allant de 10⁻⁴ à 10⁻², en particulier de 10⁻³ à 5.10⁻³ Pa.s à 20°C.

La solution peut être limpide, en particulier ne pas comprendre de particules en suspension.

Selon un autre aspect, il est décrit l'utilisation d'au moins un composé de type pyrido(3,2,1-ij)-benzoxadiazine tel que décrit ci-dessus dans la préparation d'une solution destinée à traiter ou à prévenir des infections, en particulier chez l'animal, ledit composé de type pyrido(3,2,1-ij)-benzoxadiazine étant présent en une teneur allant de 10 à 30 % en poids, notamment 11 à 28 % en poids, voire de 12 à 27 % en poids, en particulier de 13 à 25 % en poids, tout particulièrement de 14 à 23 % en poids, voire de 15 à 20 % en poids par rapport au volume total de la solution.

Selon un autre aspect, il est décrit l'utilisation d'une solution selon l'invention pour la préparation d'un médicament destiné à traiter ou à prévenir des infections, en particulier chez l'animal.

Parmi les infections susceptibles d'être traitées, on peut citer les infections du système respiratoire, du système de reproduction, du système urinaire, du système digestif, du système locomoteur, cardiovasculaire, du type cutanée, otite, ophtalmique, notamment chez les animaux.

Selon un autre aspect, il est décrit une méthode de traitement, de prévention et de métaphylaxie des infections chez des animaux. Ladite méthode de traitement peut comprendre l'injection de 0,01 à 0,5 ml de solution concentrée par kg, notamment de 0,01 à 0,1 ml/kg d'animal à traiter. En particulier le traitement peut comprendre une seule injection en un seul point.

Par exemple pour un animal de 300 kg, le traitement d'une infection respiratoire peut être réalisé avec « MARBOCYL S » avec un volume de 24 ml, administré en 2 points d'injection, et avec la solution concentrée, avec un volume de 15 ml, injecté en 1 seul point.

Par « traitement » on entend le traitement en lui-même, mais aussi la prophylaxie et la métaphylaxie.

Selon un autre aspect, il est décrit l'utilisation d'au moins un composé choisi parmi les alcools, les propylènes glycol, le polyéthylène glycol, la glycérine, en tant qu'agent stabilisant une solution d'un composé de Formule (I). Plus particulièrement, l'agent stabilisant est un alcool, notamment aromatique, alkyle, arylalkyle ou alkylaryle, en particulier comprenant de 5 à 15 atomes de carbone, et tout spécialement l'alcool benzylique et ses dérivés, en tant qu'agent stabilisant d'une solution comprenant au moins un composé de formule (I), en particulier à une forte concentration, notamment supérieure à 10 % en poids par rapport au volume total de la solution, et éventuellement un agent de solubilisation.

Selon un autre aspect, il est décrit l'utilisation d'au moins un composé choisi parmi :
- les acides minéraux, notamment l'acide chlorhydrique, bromhydrique, sulfurique, phosphorique et nitrique, et
- les acides organiques, comme les acides carboxyliques, les acides sulfoniques et les acides phosphoniques ; notamment l'acide formique, acétique, propionique, succinique, glycolique, lactique ou polylactique, malique, tartrique, citrique, ascorbique, maléique, hydroxymaléique, phénylacétique, benzoïque, 4-aminobenzoïque, anthranilique, 4-hydroxybenzoïque, salicylique, aminosalicylique, nicotinique, méthane sulfonique, éthane sulfonique, hydroxyéthane sulfonique, benzène sulfonique, p-toluènesulfonique ; les acides polycarboxyliques, comme l'acide gluconique, l'acide glucuronique, l'acide galacturonique, l'acide isophtalique et l'acide lactobionique ; les acides aminés, comme l'acide aspartique et glutamique, la méthionine, le tryptophane, la lysine, l'arginine ; leurs esters, parmi lesquels on peut citer la gluconolactone ; et leurs sels, et
- leurs mélanges,
en tant qu'agent de solubilisation d'une solution d'un composé de Formule (I), en particulier à une forte concentration, notamment supérieure à 10 % en poids par rapport au volume total de la solution, comprenant éventuellement en outre un agent stabilisant.

L'utilisation d'un agent stabilisant, et en particulier d'alcool benzylique, peut permettre en outre aux solutions concentrées en composé de formule (I), notamment en Marbofloxacine, et comprenant au moins un agent de solubilisation, notamment de la gluconolactone, de présenter un pH et/ou une osmolarité moindre par rapport aux solutions comprenant une concentration équivalente en composé de formule (I) et éventuellement en agent de solubilisation. Ceci peut en particulier permettre aux solutions concentrées comprenant au moins un composé de formule (I), un agent de solubilisation et un agent stabilisant de présenter un pH et/ou une osmolarité proche des compositions moins concentrées, notamment en composé de formule (I) et/ou en agent de solubilisation. Ceci est lié à la présence d'un agent stabilisant qui peut permettre d'utiliser moins d'agent de solubilisation.

En d'autres termes, l'addition d'agent stabilisant rend également possible la fabrication de certaines solutions très concentrées en composé de formule (I) adaptées à l'injection, qui ne le seraient pas, ou qui le seraient difficilement, sans l'ajout de cet agent. Effectivement, l'effet stabilisant peut augmenter avec la teneur en agent stabilisant.

Selon un autre aspect, il est décrit l'utilisation de l'association d'une part d'alcool benzylique et d'autre part de l'acide lactique, l'acide gluconique et/ou la gluconolactone en tant qu'agent solubilisant et stabilisant d'une solution de composé de formule (I), en particulier à une concentration supérieure à 10 %, notamment supérieure à 11 %, en particulier supérieure à 12 % en poids, tout particulièrement supérieure à 13 % en poids, voire supérieure à 14 % en poids par rapport au volume de la solution.

Dans des exemples de modes de réalisation, il est décrit :
**A.** une solution anti-infectieuse comprenant de 10 à 30 % en poids par rapport au volume total de la solution d'au moins un composé de type pyrido(3,2,1-ij)-benzoxadiazine répondant à la formule (I) suivante : 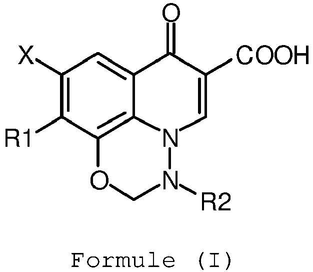
   - X représente un atome d'hydrogène, d'halogène ou une fonction hydroxy, et en particulier un atome de fluor,
   - R1 représente :
      - un radical 1-pipérazinyle, qui peut être substitué en position 4 par un groupe méthyle, acétyle ou 4-aminobenzyle ;
      - un radical morpholino ;
      - un radical 1-pyrrolidinyle substitué en position 3 par un atome de chlore ou par un groupe amino, aminométhyle, (méthylamino) méthyle, (éthylamino) méthyle ou méthoxy ;
      - un radical 1-imidazolyle qui peut être substitué en position 4 par le groupe méthyle ou
      - un radical 1-pipéridyle substitué en position 4 par un groupe hydroxy ou méthoxy, et
   - R2 représente un radical alkyle, linéaire, ramifié ou cyclique, comprenant de 1 à 10 atomes de carbone, notamment un groupe méthyle, ou
   au moins un de ses sels pharmaceutiquement acceptables, et au moins un agent de solubilisation, dans un support pharmaceutiquement acceptable.
**B.** une solution selon le mode de réalisation **A** ci-dessus caractérisée en ce que le composé répond à la formule (I) dans laquelle R1 représente un radical 1-pipérazinyle substitué en position 4 par un groupe méthyle, R2 représente un groupement méthyle et X représente un atome de fluor.
**C.** une solution selon le mode de réalisation **A** ou **B,** caractérisée en ce qu'elle comprend une teneur en composé de formule (I) allant de 11 à 28 % en poids, en particulier allant de 12 à 27 % en poids, tout particulièrement allant de 13 à 25 % en poids, encore plus particulièrement allant de 14 à 23 % en poids, voire allant de 15 à 20 % en poids par rapport au volume total de la solution.
**D.** une solution selon l'un quelconque des modes de réalisation **A** à **C,** caractérisée en ce que l'agent de solubilisation est choisi dans le groupe comprenant les acides minéraux, les acides organiques, leurs esters et leurs sels.
**E.** une solution selon l'un quelconque des modes de réalisation **A** à **D**, caractérisée en ce que l'agent de solubilisation est choisi dans le groupe comprenant l'acide lactique, l'acide gluconique et la gluconolactone.
**F.** une solution selon l'une quelconque des modes de réalisation **A** à **E**, caractérisée en ce qu'elle comprend une teneur en agent de solubilisation allant de 4 à 58 % en poids, notamment de 5 à 35 % en poids, en particulier allant de 6 à 25 % en poids, voire allant de 7 à 20 % en poids par rapport au volume total de la solution.
**G.** une solution selon l'un quelconque des modes de réalisation **A** à **F**, caractérisée en ce que le rapport molaire agent de solubilisation/composé de type pyrido(3,2,1-ij)-benzoxadiazine va de 0,9 à 4, notamment de 1 à 3, et en particulier de 1,1 à 2,4.
**H.** une solution selon l'un quelconque des modes de réalisation **A** à **G**, caractérisée en ce que l'agent de solubilisation est présent en une teneur telle que le pH de la solution va de 2 à 7, notamment de 2,8 à 5.
**I.** une solution selon l'un quelconque des modes de réalisation **A** à **H**, caractérisée en ce qu'elle comprend en outre un agent stabilisant, notamment choisi dans le groupe comprenant les alcools, les propylènes glycol, le polyéthylène glycol, la glycérine, en particulier un alcool, et tout particulièrement l'alcool benzylique et ses dérivés.
**J.** une solution selon l'un quelconque des modes de réalisation **A** à **I**, caractérisée en ce qu'elle comprend une teneur en agent stabilisant allant de 0,2 à 20 % en poids, notamment de 0,3 à 10 % en poids, en particulier de 0,4 à 5 % en poids, et tout particulièrement de 0,5% à 3 % en poids par rapport au volume total de la solution.
**K.** une solution selon l'un quelconque des modes de réalisation **A** à **J**, caractérisée en ce qu'elle comprend un rapport molaire agent stabilisant/composé de type pyrido(3,2,1-ij)-benzoxadiazine allant de 0,02 à 7, notamment de 0,05 à 5, en particulier de 0,1 à 1,5, voire de 0,2 à 1.
**L.** une solution selon l'un quelconque des modes de réalisation **A** à **K**, caractérisée en ce qu'elle présente un pH allant de 2 à 7 et notamment de 2,8 à 5.
**M.** une solution selon l'un quelconque des modes de réalisation **A** à **L**, destinée à être injectée, notamment chez des animaux, notamment des animaux de rente et/ou des animaux de compagnie.
**N.** l'utilisation d'au moins un composé de type pyrido(3,2,1-ij)-benzoxadiazine tel que décrit dans le mode de réalisation **A** ou **B** dans la préparation d'une solution destinée à traiter ou à prévenir des infections, en particulier chez l'animal, ledit composé de type pyrido(3,2,1-ij)-benzoxadiazine étant présent en une teneur allant de 10 à 30 % en poids, notamment de 11 à 28 % en poids, voire de 12 à 27 % en poids, en particulier de 13 à 25 % en poids, tout particulièrement de 14 à 23 % en poids, voire de 15 à 20 % en poids par rapport au volume total de la solution.
**O**. l'utilisation d'au moins un composé choisi parmi les alcools, les propylènes glycol, le polyéthylène glycol, la glycérine, et plus particulièrement un alcool, notamment aromatique, alkyle, arylalkyle ou alkylaryle, en particulier comprenant de 5 à 15 atomes de carbone, et tout spécialement l'alcool benzylique, en tant qu'agent stabilisant d'une solution comprenant au moins un composé de formule (I) et éventuellement un agent de solubilisation.
**P.** l'utilisation d'au moins un composé choisi parmi :
   - les acides minéraux, notamment l'acide chlorhydrique, bromhydrique, sulfurique, phosphorique et nitrique, et
   - les acides organiques, comme les acides carboxyliques, les acides sulfoniques et les acides phosphoniques ; notamment l'acide formique, acétique, propionique, succinique, glycolique, lactique ou polylactique, malique, tartrique, citrique, ascorbique, maléique, hydroxymaléique, phénylacétique, benzoïque, 4-aminobenzoïque, anthranilique, 4-hydroxybenzoïque, salicylique, aminosalicylique, nicotinique, méthane sulfonique, éthane sulfonique, hydroxyéthane sulfonique, benzène sulfonique, p-toluènesulfonique ; les acides polycarboxyliques, comme l'acide gluconique, l'acide glucuronique, l'acide galacturonique, l'acide isophtalique et l'acide lactobionique ; les acides aminés, comme l'acide aspartique et glutamique, la méthionine, le tryptophane, la lysine, l'arginine ; leurs esters, parmi lesquels on peut citer la gluconolactone ; et leurs sels, et
   - leurs mélanges,
   en tant qu'agent de solubilisation d'une solution d'un composé de Formule (I), comprenant éventuellement en outre un agent stabilisant.

Les exemples suivants sont donnés à titre illustratif et ne peuvent en aucun cas servir à limiter la portée de l'invention.

### EXEMPLES

### Exemple 1 comparatif:

Les solutions décrites dans le tableau 1 suivant ont été préparées.

| | **Lot 1** | **Témoin** |
|---|---|---|
| Marbofloxacine (en gramme) | 15, 00 | 10,00 |
| Gluconolactone (en gramme) | 8,11 | 8,00 |
| Eau déminéralisée qsp | 100 ml | 100 ml |

Des porcs de 70-80 Kg ont reçu une injection unique de solution témoin dans l'encolure droite et d'une solution du lot 1 dans l'encolure gauche, par voie intramusculaire, à la dose de 8 mg/Kg. La tolérance locale (lésions post-mortem) a été examinée 1 semaine après administration.

L'injection avec le lot 1 présente un volume de lésion d'environ 9 cm³ alors que l'injection avec le témoin montre un volume de lésion de 32 cm³.

Ceci démontre une meilleure tolérance locale de la solution concentrée en Marbofloxacine, par rapport à la solution témoin.

### Exemple 2 :

Les lots suivants ont été préparés :

| | Lot 1 | Lot 2 | Lot 3 | Lot 4 |
|---|---|---|---|---|
| Marbofloxacine | 20,00 g | 20,00 g | 20,00 g | 20,00 g |
| Gluconolactone | 19,00 g | 19,00 g | 17,00 g | 17,00 g |
| Alcool benzylique | - | 2,00 g | - | 2,00 g |
| Eau déminéralisée | qsp 100 ml | qsp 100 ml | qsp 100 ml | qsp 100 ml |
| **Stabilité à froid** | **24 heures** | **> 7 jours*** | **24 heures** | **> 7 jours*** |

| | | | | |
|---|---|---|---|---|
| * Apres 7 jours, aucun précipité n'a été observé | | | | |

Par « stabilité à froid », on entend le temps pour qu'il se forme un précipité lorsque les lots sont laissés immobiles entre +4 et +8°C.

Ceci démontre bien une amélioration de la stabilité des solutions selon l'invention.

## Revendications

1. Solution aqueuse anti-infectieuse injectable comprenant :
(i) de 15 à 20 % en poids par rapport au volume total de la solution d'au moins un composé répondant à la formule (I) suivante : 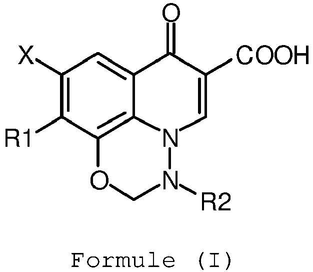
- X représente un atome d'hydrogène, d'halogène ou une fonction hydroxy,
- R1 représente :
- un radical 1-pipérazinyle, qui peut être substitué en position 4 par un groupe méthyle, acétyle ou 4-aminobenzyle ;
- un radical morpholino ;
- un radical 1-pyrrolidinyle substitué en position 3 par un atome de chlore ou par un groupe amino, aminométhyle, (méthylamino) méthyle, (éthylamino) méthyle ou méthoxy ;
- un radical 1-imidazolyle qui peut être substitué en position 4 par le groupe méthyle ou
- un radical 1-pipéridyle substitué en position 4 par un groupe hydroxy ou méthoxy, et
- R2 représente un radical alkyle, linéaire, ramifié ou cyclique, comprenant de 1 à 10 atomes de carbone, ou au moins un de ses sels pharmaceutiquement acceptables,
(ii) un agent stabilisant choisi dans le groupe comprenant les alcools, les propylènes glycol, le polyéthylène glycol et la glycérine, et
(iii) au moins un agent de solubilisation choisi dans le groupe comprenant les acides minéraux, les acides organiques et leurs esters, et leurs mélanges,
dans un support pharmaceutiquement acceptable.

2. Solution selon la revendication 1 dans laquelle le composé répond à la formule (I) dans laquelle R1 représente un radical 1-pipérazinyle substitué en position 4 par un groupe méthyle, R2 représente un groupement méthyle et X représente un atome de fluor.

3. Solution selon la revendication 1 ou 2, dans laquelle l'agent de solubilisation est choisi dans le groupe comprenant l'acide lactique, l'acide gluconique et la gluconolactone.

4. Solution selon l'une quelconque des revendications 1 à 3, laquelle comprend une teneur en agent de solubilisation allant de 4 à 58 % en poids, par rapport au volume total de la solution.

5. Solution selon l'une quelconque des revendications 1 à 4, dans laquelle le rapport molaire agent de solubilisation/composé de formule (I) va de 0,9 à 4.

6. Solution selon l'une quelconque des revendications 1 à 5, dans laquelle l'agent de solubilisation est présent en une teneur telle que le pH de la solution va de 2 à 7.

7. Solution selon l'une quelconque des revendications 1 à 6, laquelle comprend une teneur en agent stabilisant allant de 0,2 à 20 % en poids par rapport au volume total de la solution.

8. Solution selon l'une quelconque des revendications 1 à 7, laquelle comprend un rapport molaire agent stabilisant/composé de formule (I) allant de 0,02 à 7.

9. Solution selon l'une quelconque des revendications 1 à 8, laquelle présente un pH allant de 2 à 7.

10. Solution selon l'une quelconque des revendications 1 à 9 pour son utilisation dans le traitement ou la prévention des infections chez l'animal.

## Patentansprüche

1. Wässrige anti-infektiöse Injektionslösung, umfassend:
**(i)** 15 bis 20 Gew.-%, bezogen auf das Gesamtvolumen der Lösung, mindestens einer Verbindung, die der folgenden Formel (I) entspricht: 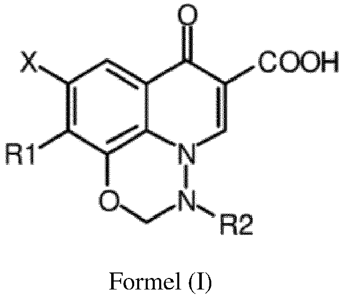 wobei:
- X ein Wasserstoffatom, ein Halogenatom oder eine Hydroxyfunktion repräsentiert,
- R1 repräsentiert:
• einen 1-Piperazinylrest, der in Position 4 durch eine Methyl-, Acetyl- oder 4-Aminobenzylgruppe substituiert sein kann;
• einen Morpholinorest;
• einen 1-Pyrrolidinylrest, der in Position 3 durch ein Chloratom oder eine Amino-, Aminomethyl-, (Methylamino)methyl-, (Ethylamino)methyl- oder Methoxygruppe substituiert ist;
• einen 1-Imidazolylrest, der in Position 4 durch die Methylgruppe substituiert sein kann, oder
• einen 1-Piperidylrest, der in Position 4 durch eine Hydroxy- oder Methoxygruppe substituiert ist, und
- R2 einen linearen, verzweigten oder cyclischen Alkylrest repräsentiert, der 1 bis 10 Kohlenstoffatome umfasst, oder
mindestens eines ihrer pharmazeutisch annehmbaren Salze,
**(ii)** ein Stabilisierungsmittel, ausgewählt aus der Gruppe umfassend Alkohole, Propylenglykole, Polyethylenglykol und Glycerin, und
**(iii)** mindestens einen Lösungsvermittler, ausgewählt aus der Gruppe umfassend Mineralsäuren, organische Säuren und ihre Ester und ihre Gemische,
in einem pharmazeutisch annehmbaren Träger.

2. Lösung nach Anspruch 1, wobei die Verbindung der Formel (I) entspricht, wobei R1 einen 1-Piperazinylrest darstellt, der in Position 4 durch eine Methylgruppe substituiert ist, R2 eine Methylgruppe darstellt und X ein Fluoratom darstellt.

3. Lösung nach Anspruch 1 oder 2, wobei der Lösungsvermittler ausgewählt ist aus der Gruppe umfassend Milchsäure, Gluconsäure und Gluconolacton.

4. Lösung nach einem der Ansprüche 1 bis 3, die einen Lösungsvermittlergehalt im Bereich von 4 bis 58 Gew.-%, bezogen auf das Gesamtvolumen der Lösung, umfasst.

5. Lösung nach einem der Ansprüche 1 bis 4, wobei das Molverhältnis von Lösungsvermittler/Verbindung der Formel (I) im Bereich von 0,9 bis 4 liegt.

6. Lösung nach einem der Ansprüche 1 bis 5, wobei der Lösungsvermittler in einem solchen Gehalt vorliegt, dass der pH-Wert der Lösung im Bereich von 2 bis 7 liegt.

7. Lösung nach einem der Ansprüche 1 bis 6, die einen Gehalt an Stabilisierungsmittel im Bereich von 0,2 bis 20 Gew.-%, bezogen auf das Gesamtvolumen der Lösung, umfasst.

8. Lösung nach einem der Ansprüche 1 bis 7, die ein Molverhältnis von Stabilisierungsmittel/Verbindung der Formel (I) im Bereich von 0,02 bis 7 umfasst.

9. Lösung nach einem der Ansprüche 1 bis 8, die einen pH-Wert im Bereich von 2 bis 7 aufweist.

10. Lösung nach einem der Ansprüche 1 bis 9 zur Verwendung in der Behandlung oder Prävention von Infektionen bei Tieren.

## Claims

1. An aqueous anti-infective injectable solution comprising:
(i) from 15 to 20% by weight based on the total volume of the solution of at least one compound having the following formula (I) : 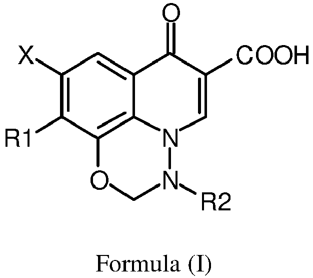
- X represents a hydrogen atom, a halogen atom or a hydroxy function,
- R1 represents :
• a 1-piperazinyl radical which may be substituted in the 4-position with methyl, acetyl or 4-aminobenzyl group;
• a morpholino radical;
• a 1-pyrrolidinyl radical substituted in the 3-position with a chlorine atom or by an amino, aminomethyl, (methylamino)methyl, (ethylamino)methyl or methoxy group;
• a 1-imidazolyl radical which may be substituted in the 4-position with a methyl groupe or
• a 1-piperidyl radical substituted in the 4-position with a hydroxy or methoxy group, and
- R2 represents a linear, branched or cyclic alkyl radical containing from 1 to 10 carbon atoms, or
at least one of its pharmaceutically acceptable salts,
(ii) a stabilizing agent selected from the group comprising alcohols, propylene glycols, polyethylene glycol and glycerine, and
(iii) at least one solubilizing agent selected from the group comprising mineral acids, organic acids and esters thereof, and mixtures thereof,
in a pharmaceutically acceptable carrier.

2. Solution according to claim 1 wherein the compound has formula (I) in which R1 represents a 1-piperazinyl radical substituted in the 4-position with a methyl group, R2 represents a methyl group and X represents a fluorine atom.

3. A solution according to claim 1 or 2, wherein the solubilizing agent is selected from the group comprising lactic acid, gluconic acid and gluconolactone.

4. A solution according to any one of Claims 1 to 3, wherein the content of solubilizing agent ranges from 4 to 58% by weight, based on the total volume of the solution.

5. A solution according to any one of Claims 1 to 4, wherein the molar ratio solubilizing agent/compound of formula (I) is from 0.9 to 4.

6. A solution according to any one of claims 1 to 5, wherein the solubilizing agent is present in an amount such that the pH of the solution is from 2 to 7.

7. A solution according to any one of Claims 1 to 6, wherein the content of stabilizing agent ranges from 0.2 to 20% by weight based on the total volume of the solution.

8. A solution according to any one of Claims 1 to 7, which comprises a molar ratio stabilizing agent /compound of formula (I) from 0.02 to 7.

9. A solution according to any one of Claims 1 to 8 which has a pH of from 2 to 7.

10. A solution according to any one of claims 1 to 9 for use in the treatment or prevention of infections in animals.
